# EUROPEAN PATENT SPECIFICATION

(11) **EP 0 903 365 B1**
(45) Date of publication and mention of the grant of the patent: **29.01.2003**
(21) Application number: 98204328.3
(22) Date of filing: 14.07.1994
(51) Int. Cl.: C08G 65/10, C08G 65/20, C08G 65/04, C08G 65/16

(54) **Polymerization of cyclic ethers using heterogeneous catalysts**
Polymerisierung von Polyethern in Gegenwart von heterogenen Katalysatoren
Polymérisation de polyéthers utilisant des catalyseurs hétérogènes

(30) Priority: 16.07.1993 US 93119
(43) Date of publication of application: 24.03.1999
(62) Divisional of application: 94923920.6
(73) Proprietor: E.I. DU PONT DE NEMOURS AND COMPANY, Wilmington Delaware 19898 (US)
(72) Inventor: Drysdale, Neville Everton, Newark, Delaware 19702-1026 (US); Herron, Norman, Newark, Delaware 19711 (US)
(74) Representative: Jones, Alan John

(56) References cited:
- EP-A- 0 352 856
- WO-A-94/19392
- US-A- 3 842 019
- US-A- 3 864 287

## Description

### FIELD OF THE INVENTION

Polymerization of cyclic ethers to polyethers is catalyzed by heterogeneous catalysts which contain selected metal cations. The metal cation may be the charge balancing counterion of a zeolite structure, or a metal perfluoroalkylsulfonate, preferably a triflate, attached to a support may be used. Depolymerization of polytetrahydrofurans to tetrahydrofurans is catalyzed by a metal perfluoroalkylsulfonate, preferably a triflate, attached to a support.

### TECHNICAL BACKGROUND

Cyclic ethers are polymerized by various means to give products of widespread utility. For instance, ethylene oxide is polymerized to polyethylene oxide which is useful for (in lower molecular weight grades) ceramics, cosmetics, lubricants, polyurethanes, (and in higher molecular weight grades), packaging film, denture adhesives, lubricants, and flocculation, and tetrahydrofuran (THF) is polymerized to poly(tetramethylene ether) glycol which is useful in the preparation of Spandex fibers, polyurethane resins useful in elastomeric parts, and thermoplastic elastomers useful for molding various mechanical parts. Therefore, improved methods of making these polymers are sought.

U.S. Patent 4,303,782 describes the use of zeolites to catalyze the polymerization of tetrahydrofuran. These polymerization appear to proceed very slowly.

U.S. Patent 3,842,019 describes the polymerization of oxiranes and other small ring compounds by a presumed cationic mechanism, using as the catalyst the decomposition products of metal perfluoroalkylsulfonates. These catalysts are described as "latent", that is no reaction occurs until the metal salt is decomposed. The reactions reported are relatively slow, even at elevated temperatures.

U.S. Patents 5,084,586 and 5,124,417 describe the cationic polymerization of various monomers, including cyclic ethers, using onium cations, whose corresponding anions are fluoroalkylsulfatometallates. Onium ion catalyzed cationic polymerizations are well known, and there is no mention in these patents of the use of metal salts not containing onium ions, such as metal triflates, as catalysts for the polymerization of cyclic ethers.

Japanese Patent Application 51-82397 describes the polymerization of tetrahydrofuran using a combination of fluorosulfonic acid and a carboxylic acid as catalysts. No mention is made of metal salts, such a metal triflates as catalysts.

J. S. Hrkach, et al., Macromolecules, vol. 23, p. 4042-4046 (1990) describe the polymerization of tetrahydrofuran using trimethylsilyl trifluoromethanesulfonate (herein triflate) as the initiator. No mention is made of any other triflates as catalysts for this polymerization.

S. L. Borkowsky, et al., Organometal., vol. 10, p. 1268-1274 (1991) report that certain zirconium complexes can initiate the polymerization of tetrahydrofuran. No mention is made of zirconium perfluoroalkylsulfonates, or of copolymers.

T. Misaki, et al., Nippon Kagaku Kaishi, p. 168-174 (1973) report on the polymerization of THF using a combination of metal aceylacetonates and acetyl chloride. None of the metal compounds herein disclosed as catalysts are mentioned.

Japanese Patent Application 51-82397 describes the polymerization of tetrahydrofuran using a combination of fluorosulfonic acid and a carboxylic acid as catalysts. No mention is made of metal salts as catalysts.

### SUMMARY OF THE INVENTION

This invention concerns a process for the polymerization of cyclic ethers, comprising, contacting one or more oxetanes, tetrahydrofurans, oxepanes, 1,3-dioxolanes, or 1,3,5-trioxanes; with a zeolite which contains a metal cation selected from the group consisting of strontium, barium, scandium, yttrium, the rare earth metals, titanium, zirconium, hafnium, vanadium, niobium, chromium, molybdenum, tantalum, tungsten, rhenium, iron, ruthenium, osmium, rhodium, iridium, palladium, copper, platinum, silver, gold, zinc, cadmium, mercury, aluminum, gallium, indium, thulium, germanium, tin, lead, arsenic, antimony and bismuth; and an accelerator selected from the group consisting of carboxylic anhydrides, acyl halides and carboxylic acids whose pKa in water is less than about 6.

This invention also concerns a process for the polymerization of cyclic ethers, comprising, contacting one or more oxiranes, oxetanes, tetrahydrofurans, oxepanes, 1,3-dioxolanes, or 1,3,5-trioxanes; with a heterogeneous catalyst containing a metal perfluoroalkylsulfonate attached to the surface of said catalyst, said metal selected from the group consisting of strontium, barium, scandium, yttrium, the rare earth metals, titanium, zirconium, hafnium, vanadium, niobium, chromium, molybdenum, tantalum, tungsten, rhenium, iron, ruthenium, osmium, rhodium, iridium, palladium, copper, platinum, silver, gold, zinc, cadmium, mercury, aluminum, gallium, indium, thulium, germanium, tin, lead, arsenic, antimony and bismuth; and an accelerator selected from the group consisting of carboxylic anhydrides, acyl halides, and carboxylic acids whose pKa in water is less than about 6.

This invention also concerns a process for the depolymerization of a polyether to a tetrahydrofuran, comprising, contacting at a temperature of about 100°C to about 250°C, a polymer consisting essentially of one or more repeat units of the formula

-[CHR¹CR²R³CR²R³CHR⁴O]-

wherein each R¹, R², R³ and R⁴ is independently hydrogen or hydrocarbyl containing 1 to 20 carbon atoms, with a heterogeneous catalyst containing a metal perfluoroalkylsulfonate attached to the surface of said catalyst, said metal selected from the group consisting of strontium, barium, scandium, yttrium, the rare earth metals, titanium, zirconium, hafnium, vanadium, niobium, chromium, molybdenum, tantalum, tungsten, rhenium, iron, ruthenium, osmium, rhodium, iridium, palladium, platinum, copper, silver, gold, zinc, cadmium, mercury, aluminum, gallium, indium, thulium, germanium, tin, lead, arsenic, antimony and bismuth.

### DETAILS OF THE INVENTION

All of the descriptions of the processes that follow, except for those of the catalysts themselves, and where otherwise noted, are applicable to both types of heterogeneous catalysts, the zeolites and those to which metal perfluoroalkylsulfonates are attached.

In the polymerization processes described herein one or more cyclic ethers, oxiranes, oxetanes, tetrahydrofurans, oxepanes, 1,3-dioxolanes, or 1,3,5-trioxanes are polymerized to form a polyether. Oxirane (more commonly called epoxide) is herein given its usual structure, a saturated three membered ring containing two carbon atoms and one oxygen atom. Oxetane is also given its common meaning, a saturated four membered ring containing 3 carbon atoms and one oxygen atom. The term 1,3-dioxolane means a saturated 5 membered ring which contains two oxygen atoms separated by 1 carbon atom. The term 1,3,5-trioxane means a six membered ring containing 3 oxygen atoms in which the oxygen atoms and carbons atoms are alternated.

The terms oxirane, oxetane, oxepane, 1,3-dioxolane, 1,3,5-trioxane, and tetrahydrofuran include compounds containing those ring systems which are substituted with hydrocarbyl or hydrocarbylene groups containing 1 to 20 carbon atoms. The hydrocarbylene groups form carbocyclic rings, which include bicyclic, tricyclic, etc. systems. By a hydrocarbylene group herein is meant a divalent radical containing carbon and hydrogen which is part of a carbocyclic ring.

Preferred cyclic ethers have the formula 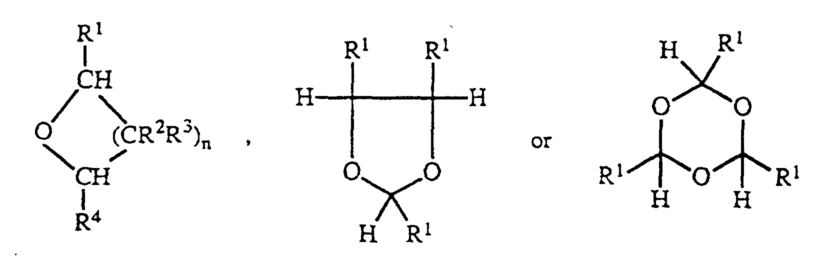 wherein n is 2 or 4, and each R¹, R², R³ and R⁴ is independently hydrogen or hydrocarbyl containing 1 to 20 carbon atoms. Some cyclic ethers polymerize to give repeat units of the formula -[CHR¹(CR²R³)ₙCHR⁴O]-. In a more preferred cyclic ether all of R¹, R², R³ and R⁴ are hydrogen. In another more preferred cyclic ether where n=2, R¹, one of R², both of R³ and R⁴ are hydrogen, and the remaining R² is alkyl containing 1-4 carbon atoms, especially preferably the remaining R² is methyl. By hydrocarbyl herein is meant a univalent radical containing carbon and hydrogen.

The polymerization is carried out in the presence of an accelerator (sometimes also called a co-catalyst). Suitable accelerators are carboxylic anhydrides, acyi halides and carboxylic acids whose pKa is less than about 6 in water.

By a carboxylic anhydride is meant a compound containing the grouping -C(O)O(O)C-, wherein the free valencies are to other carbon atoms. A preferred carboxylic anhydride is an anhydride of an alkyl carboxylic acid or a halogen substituted alkyl carboxylic acid, and particularly preferred anhydrides are acetic anhydride and trifluoroacetic anhydride.

By an acyl halide is meant a compound containing the grouping -C(O)X, where X is chlorine or bromine and the free valence it to another carbon atom. In preferred acyl halides X is chlorine. Preferred acyl halides are alkyl acyl halides, and especially preferred are acetyl halides, more preferably acetyl chloride.

By a carboxylic acid is meant a compound containing the grouping -C(O)OH, wherein the free valence is to another carbon atom. Preferred carboxylic acids have a pKa of less than 5 in water. Useful carboxylic acids include, but are not limited to formic, acetic, trifluoroacetic, chloroacetic, benzoic, trichloroacetic, p-nitrobenzoic, butyric, and naphthoic acids. Preferred carboxylic acids are trifluoroacetic, formic, acetic, cyanoacetic, nitropropionic, nitrobenzoic, acrylic and methacrylic. These and other acids that themselves don't cause polymerization of the cyclic ethers are also believed to be accelerators, especially if their pKa in water is about 6 or less.

When carboxylic anhydride is present one half or more of the end groups are carboxylic esters. As is known to the artisan, these may be hydrolyzed to hydroxyl groups by reaction with water, preferably in the presence of a catalyst, such as a strong acid (sulfuric acid for instance) or a strong base (such as NaOH). The proportion of acetate ends increases the longer the polymerization is allowed to proceed. Although the polymeric diol is often the desired product (it can be used to make other polymers, such as polyurethanes and polyesters), the half ester or diester is also useful, as in relatively low molecular weight polymers which can be used as solvents.

When acyl halides are used as the accelerator, the end groups are usually ester on one end, and the halide, X, on the other. Thus the complete formula for such a polymer could be X-[CHR¹(CR²R³)ₙCHR⁴O]-C(O)Y, where Y is the group to which the acyl group of the acyl halide was bound. Such polymers are useful as intermediates for the preparation of polymers containing different functional groups. For example, the ester may be hydrolyzed to a hydroxyl group, and the halide may be reacted to form another functional group such as nitrile. If a bis(acyl halide), X(O)CYC(O)X, is used as the accelerator, the product of the polymerization will be a polyether with halide (X) end groups which contains two internal ester groups, and may have the formula X- [CHR¹(CR²R³)ₙCHR⁴O]-C(O)YC (O) - [OCHR¹(CR²R³)ₙCHR⁴]-x. Useful bis(acyl halides) include adipoyl chloride, terephthaloyl chloride, and diglycolyl chloride [Cl(O)CCH₂OCH₂C(O)Cl].

When a carboxylic acid is used as the accelerator, the end groups are usually mostly ester. Thus the complete formula for such a polymer could be Y-C(O)-O-[CHR¹(CR²R³)ₙCHR⁴O]-C(O)Y, where Y is the group to which the acyl group of the carboxylic acid was bound. The ester group may be hydrolyzed as described above in the paragraph describing the products when a carboxylic anhydride is used as the accelerator.

The polymerization may be run at a temperature of about -80°C to about 130°C. If this temperature is above the boiling point of the cyclic ether monomer, a pressure vessel may be used. A preferred temperature is ambient to the boiling point of the monomer or 110°C, whichever is lower. An inert solvent such as di-n-butyl ether, diethyl ether or toluene may be used, but it is preferred if solvents are not present. Protic compounds such as water, methanol and ethanol should preferably not be present, and it is convenient to exclude them by drying the starting materials and keeping the process under an inert dry gas such as nitrogen or dry air. As in most chemical processes, the ingredients should be mixed. Continued agitation is preferred to assure contact of the process liquids with the heterogeneous catalyst, and to avoid overheating. The polymerization is mildly exothermic. If the polymerization temperature goes up appreciably, refluxing of the monomer may be used to help cool the process.

With both types of catalysts the polymerization may be run in a variety of methods, such as batch, semi-continous, or continuous. While the heterogeneous catalyst may be recovered each time, as by filtration, and reused, in another embodiment the catalyst is fixed in place and the polymerization mass circulated or agitated so that uniform contact with the catalyst surface is obtained. In this way, the catalyst may be used for long periods in a continuous process, or for many batches in a batch polymerization, without the need to recover the catalyst. Contact time of the liquid reaction mass with the catalyst will depend on many factors, such as the catalytic metal used, its concentration on the catalyst, the temperature, cyclic ether being polymerized, etc., but will usually be in the range of a few minutes to a few hours.

Both types of catalysts used herein contain selected metal cations. Preferred metal cations are those of strontium, scandium, yttrium, the rare earth metals, titanium, zirconium, hafnium, vanadium, niobium, tantalum, chromium, molybdenum, tungsten, rhenium, iron, ruthenium, palladium, copper, gold, zinc, tin and bismuth. More preferred metals are yttrium, the rare earth metals, scandium and zirconium. Especially preferred metals are yttrium, ytterbium, dysprosium, erbium, neodymium, lanthanum, scandium and zirconium. Another preferred metal is "mischmetall" (sometimes also called "didymium"), which is a mixture of rare earth metals as obtained from the ore. By rare earths herein is meant lanthanum, cerium, praeseodymium, neodymium, promethium, samarium, europium, gadolinium, terbium, dysprosium, holmium, erbium, thulium, ytterbium, and lutetium. By a perfluoroalkylsulfonate herein is meant a metal salt of a perfluoroalkylsulfonate in which the metal is bonded to one or more perfluoroalkylsulfonate groups.

One of the catalysts used herein is a zeolite in which some of the metal cations present are the cations listed above. The anions of such cations are not critical, and may be those normally found in zeolites. The zeolites containing the appropriate metal cations can be made by the ion exchange of cations in known zeolites. Such ion exchange processes are know to the artisan, see for instance D. W. Breck in Zeolite Molecular Sieves, R.E. Krieger Publishing Co., Malabar, Florida, 1984 and Examples 7, 9, 12, 14, 16, 18, and 20. It is preferred if at least 0.5 atom percent of the metals and metalloids in the zeolite are one of the useful "catalytic" metals, more preferably at least 5 atom percent. It has been found that the zeolite catalysts usually yield polyethers with bimodal molecular weight distributions.

In another heterogeneous catalyst, a "catalytic" metal perfluoroalkylsulfonate is attached to the surface of a material that in effect acts as a heterogeneous support for the metal perfluoroalkylsulfonate. The metal is not attached to the surface through the perfluoroalkylsulfonate, but through another bond or ligand. The catalytic metal should have at least one perfluoroalkylsulfonate anion attached to it, and preferably, except for the group attaching the metal to the support surface, all of the groups should be perfluoroalkylsulfonate.

The metal may be attached to the surface by a covalent bond, or coordination, or any other method. It is preferred if significant amounts (>25%, preferably <10%) of the catalytic metal cannot be leached from the heterogeneous catalyst by the polymerization process liquids. In one method of attachment, a ligand which can coordinate with the metal cation is attached via one or more covalent bonds to the surface of the support, and then the metal cation is coordinated to the ligand, thereby fixing the metal cation on the support surface. Particularly useful for such are silicon compounds to which the ligand is attached by a stable (to hydrolysis and the polymerization process conditions) bond, and in which the silicon is directly attached to groups which are readily hydrolyzed. When these hydrolytically unstable groups are hydrolyzed from the silicon atom, the resulting "compound" can readily bond to surfaces which have hydroxyl groups present. Many common supports, such as alumina, silica (gel), many metal oxides, and others have such surfaces. After the ligand is attached to the surface, an appropriate metal compound is contacted with the surface containing the ligands, and the metal cation becomes fixed to the support surface. See Examples 1, 3, 5, 22, 24, 26, and 28 for such processes.

The heterogeneous supports for such catalysts can be those which are commonly used for supported catalysts. It is preferred if they have a relatively large surface area, at least 25 m²/gm, and it is also preferred if the support is inorganic (inorganic includes various forms of carbon, such as activated carbon, graphite, etc.). Useful supports include alumina, silica, silica-aluminates, carbon, zirconia, yttria, magnesia, ceria, aluminum fluoride and barium sulfate. Preferred supports are alumina, silica, silica-aluminates, carbon and zirconia. It is preferred if the supports themselves are acidic. Although not critical, a convenient amount of the catalytic metal on the catalyst is about 0.1 to about 20 weight percent, measured as catalytic metal.

The depolymerization process is carried out at about 100°C to about 250°C, preferably about 130°C to about 200°C. Although air can be used to blanket the process, it is preferred to use an inert atmosphere such as nitrogen to avoid possible side reactions. A solvent may be used, but it is preferred to carry out the process without solvent.

The amount of catalyst compared to polyether present is not critical, 0.1-15% by weight (percent of the catalyst to polyether) being useful, preferably about 1 to 3% by weight of catalyst.

The depolymerization process may be carried out by just heating the polyether in the presence of the heterogeneous catalyst. In order to avoid boiling off the often volatile tetrahydrofurans, a pressure vessel may be needed. However, it is preferred to carry out the depolymerization while constantly distilling off the (substituted) tetrahydrofuran as it forms. It is believed that this ensures driving this process to produce the monomeric tetrahydrofuran.

In both the polymerization and depolymerization processes disclosed herein the heterogeneous catalyst may be recovered and reused in either process. It may be recovered from both processes by filtration, and if desired, drying. The recovered catalyst may be used again in the polymerization or depolymerization processes.

All of the above processes may be carried out as batch, semibatch or continuous processes. For all of the processes, continuous type processes are preferred.

In the Examples, the following abbreviations are used:
- ACA -: acetic anhydride
- DETM -: diethyl 2-[3-(triethoxysilyl)propyl)]-malonate
- GPC -: gel permeation chromatography
- Mn -: number average molecular weight
- Mw -: weight average molecular weight
- PD -: polydispersity, Mw/Mn
- PS -: polystyrene
- SS -: stainless steel
- STD -: standard
- THF -: tetrahydrofuran

### EXAMPLE 1

### Heterogeneous 10 wt% Yttrium triflate on alumina catalyst preparation

25 g commercial alumina pellets AL-3945 (3.2 mm dia x 3.2 mm) was placed in 250 mL water and the pH adjusted to 3 with acetic acid. After stirring 15 mins the pellets were collected by filtration and suction dried. A solution of 190 mL ethanol and 10 mL water had its apparent pH adjusted to 5 with acetic acid and 5 g of DETM was added. After stirring for 5 minutes the alumina pellets were added and agitated for 30 minutes. The supernatant liquid was then decanted and the pellets washed with 2 x 25 mL ethanol and suction dried. The solid was dried in flowing nitrogen by heating to 110°C for 1 hour and then sealed and taken into a nitrogen glove box. 5 g yttrium triflate was dissolved in 50 mL acetonitrile and added to the dry alumina pellets. This slurry was allowed to sit undisturbed overnight in the glove box and then evaporated to dryness in vacuo. The solid was then extracted with 2 x 25 mL acetonitrile, filtered and washed with acetonitrile. Evaporation of all washings and extracts indicated that ∼50% of the original yttrium triflate had been retained on the pellets. The pellets were suction dried and then dried in flowing nitrogen at 110°C for 1 hour before returning to the glove box for collection and storage prior to testing.

### EXAMPLE 2

### Polymerization of THF over 10% Yttrium Triflate on Alumina Pellets with Acetic Anhydride

THF was charged into a 500 mL capacity ISCO pump, which was connected to a 3 way 0.3 cm SS connector ("T" mixer) via 8 cm of 0.3 cm SS tubing containing a check valve. A second ISCO pump was charged with acetic anhydride and this was connected to the "T" mixer by 75 cm of 0.3 cm SS tubing also containing a check valve. In a dry box, all of the catalyst made in Example 1 (23.9 g) was charged into the reactor.¹ This was in turn connected to the "T" mixer by 12 cm of SS tubing. This reactor was then connected to a holdup tank (approximately 60 mL volume) via Cajon flex tubing with ultra torr fitting (0.6 cm, 13 cm. Polymerization was started by first filling the reactor with THF (approximately 83 mL). Then THF was fed at a rate of 0.75 mL/min and acetic anhydride at a rate of 0.075 mL/min. The exiting polymerized solution was fed to a beaker. After each fraction, the pumps were refilled and the collected polymer solution diluted with diethyl ether and washed with water, separated, concentrated at reduced pressure and then dried under vacuum. The following are the conditions under which the various fractions were collected, together with weight of polymer obtained and GPC analysis:

| Fraction | Polymer. Time | THF Flow Rate (mL/min) | ACA Flow Rate (mL/min) | Wt. (g) | Mn .(PS STD.) | Mw | PD |
|---|---|---|---|---|---|---|---|
| 1 | 372 mins. | 0.75 | 0.075 | 108.9 | 10800 | 22800 | 2.11 |
| 2 | 899 mins. | 0.50 | 0.05 | 139.2 | 15800 | 36800 | 2.32 |
| 3 | 491 mins. | 0.50 | 0.05 | 17.73 | 27400 | 92300 | 3.37 |
| 4 | 975 mins. | 0.50 | 0.05 | 74.19 | 28400 | 60200 | 2.12 |
| 5 | 464 mins. | 0.50 | 0.05 | 20.6 | | | |
| 6 | 935 mins | 0.50 | 0.05 | 38.25 | | | |
| ¹reactor design to be supplied upon preparation of patent application. | | | | | | | |

### EXAMPLE 3

### Heterogeneous 10 wt% Yttrium triflate on silica-alumina catalyst preparation

25 g commercial silica-alumina pellets (Alfa cat # 31269; 91% Al₂O₃, 6% SiO₂) was placed in 250 mL water and the pH adjusted to 3 with acetic acid. The pellets were then treated in a manner identical to that described in Example 1 above.

### EXAMPLE 4

### Polymerization of THF over 10% Yttrium on Silica-Alumina Pellets with Acetic Anhydride

All of the catalyst of Example 3, 10% yttrium triflate on silica-alumina pellets (20.8 g) was charged in the reactor. The apparatus was as described in Example 1. The following are the conditions under which polymer was collected, together with weight of polymer obtained and GPC analysis.

| Fraction | Polymer. Time | THF Flow Rate (mL/min) | ACA Flow Rate (mL/min) | Wt. (g) | Mn (PS STD.) | Mw | PD |
|---|---|---|---|---|---|---|---|
| 1 | 945 mins. | 0.50 | 0.05 | 148.78 | 4370 | 8790 | 2.01 |
| 2 | 466 mins. | 0.50 | 0.05 | 121.3 | 17000 | 33000 | 1.94 |
| 3 | 990 mins. | 0.50 | 0.05 | 177.17 | 24800 | 48900 | 1.97 |
| 4 | 449 mins. | 0.50 | 0.05 | 70.05 | 10000 | 26800 | 2.66 |

### EXAMPLE 5

### Heterogeneous 10 wt% Zirconium triflate on alumina catalyst preparation.

25 g commercial alumina pellets (Al-3945, 3.2 mm dia. x 3.2 mm)) was placed in 250 mL water and the pH adjusted to 3 with acetic acid. The pellets were then treated in a manner identical to that described in Example 1 above except substituting zirconium triflate for yttrium triflate.

### EXAMPLE 6

### Polymerization of THF over 10% Zirconium Triflate on Alumina Pellets with Acetic Anhydride

All of the catalyst of Example 5, 10% zirconium triflate on alumina pellets (22.74 g), was charged in the reactor. The apparatus was as described in Example 1. The following are the conditions under which polymer was collected, together with weight of polymer obtained.

| Fraction | Polymer. Time | THF Flow Rate (mL/min) | ACA Flow Rate (mL/min) | Wt. (g) | Mn (PS STD.) | Mw | PD |
|---|---|---|---|---|---|---|---|
| 1 | 928 mins. | 0.50 | 0.05 | 247.97 | 9010 | 22500 | 2.51 |
| 2 | 498 mins. | 0.50 | 0.05 | 75.00 | 10800 | 32500 | 2.99 |
| 3 | 948 mins. | 0.50 | 0.05 | 164.13 | 29000 | 55800 | 1.92 |
| 4 | 482 mins. | 0.50 | 0.05 | 58.91 | 45000 | 80700 | 1.79 |
| 5 | 896 mins. | 0.50 | 0.05 | 201.15 | 41100 | 80600 | 1.96 |

### EXAMPLE 7

### Preparation of La loaded zeolite HY for THF polymerization

10 g of zeolite LZY-82 (NH₄⁺ ion form of zeolite Y) was slurried into 1 liter distilled water and the pH was adjusted to 4 with nitric acid. 1.56 g lanthanum nitrate hexahydrate (calculated to give a final product having ∼5wt% La) was added and the slurry stirred and warmed for 4 hours. The stirring was stopped and the mixture left to sit overnight to complete the exchange. The solid was collected by filtration and washed with 1 liter distilled water then suction dried. The wet solid was loaded into a horizontal tube furnace and fired as follows in flowing dry air (flow rate 200 mL/min) :
Room temperature to 500°C at 12°C/min.
Hold at 500°C for 2hours
Cool to room temperature over 1hour and collect white powder.

The cool powder was quickly transferred to a tightly capped sample vial in order to minimize exposure to atmospheric moisture. A small portion of the material was sent for x-ray diffraction analysis and showed that the zeolite crystallinity had been maintained during the ion-exchange and calcination procedure and that there was no presence of a bulk La₂O₃ phase.

### EXAMPLE 8

### Polymerization of THF with Lanthanum Zeolite and Acetic Anhydride

In a dry box, the lanthanum zeolite of Exmaple 7 (2.00 g) was added to an oven dried 100 mL round bottom flask equipped with a stirring bar. The flask was sealed with a rubber septum and removed from the dry box. A nitrogen bleed was attached and THF (20.00 mL) and acetic anhydride (1.00 mL) were added. After stirring overnight the resulting material was diluted with THF (50 mL) and filtered. The resulting filtrate was concentrated at reduced pressure. The vicous liquid was dissolve in THF (50 mL) and dried over anhydrous NaHCO₃, filtered concentrated at reduced pressure and then dried under vacuum. Polymer yield: 3.86 g. GPC analysis: Mn = 969, Mw = 5970, PD = 6.17 (PS STD., bimodal distribution).

### EXAMPLE 9

### Preparation of 5% Y loaded zeolite HY for THF polymerization

10 g of zeolite LZY-82 (NH₄⁺ ion form of zeolite Y) was slurried into 1 liter distilled water and the pH was adjusted to 4 with nitric acid. 2.20 g yttrium nitrate pentahydrate (calculated to give a final product having ∼5 wt% Y) was added and the slurry stirred and warmed for 4 hours. The stirring was stopped and the mixture left to sit overnight to complete the exchange. The solid was collected by filtration and washed with 1 liter distilled water then suction dried. The wet solid was loaded into a horizontal tube furnace and fired as follows in flowing dry air (flow rate 200 mL/min) :
Room temperature to 500°C at 12°C/min.
Hold at 500°C for 2 hours.
Cool to room temperature over 1 hour and collect white powder.

The cool powder was quickly transferred to a tightly capped sample vial in order to minimize exposure to atmospheric moisture. A small portion of the material was sent for x-ray diffraction analysis and showed that the zeolite crystallinity had been maintained during the ion-exchange and calcination procedure and that there was no presence of a bulk Y₂O₃ phase.

### EXAMPLE 10

### Polymerization of THF with 5% Yttrium Zeolite and Acetic Anhydride

In a dry box, the yttrium zeolite of Example 9 (5.00 g) was added to an oven dried 100 mL round bottom flask equipped with a stirring bar. The flask was sealed with a rubber septum and removed from the dry box. A nitrogen bleed was attached and THF (20.00 mL) and acetic anhydride (1.00 mL) were added. After stirring overnight ether (50 mL was added to the resulting polymerized solution. After filtering, ether (50 mL) and water (25 mL) were added and the organic phase separated, concentrated at reduced pressure and then dried under vacuum. Polymer yield: 2.21 g. GPC analysis: Mn = 840, Mw = 4600, PD = 5.48 (PS STD., bimodal distribution).

### EXAMPLE 11

### Polymerization of THF with 5% Yttrium Zeolite HY and Acetic Anhydride

In a dry box, the 5% yttrium zeolite HY of Example 9 (10.00 g) was added to an oven dried 100 mL round bottom flask equipped with a stirring bar. The flask was sealed with a rubber septum and removed from the dry box. A nitrogen bleed was attached and THF (20.00 mL) and acetic anhydride (1.00 mL) were added. After stirring overnight ether (50 mL) was added to the resulting polymerized solution. After filtering, ether (50 mL) and water (25 mL) were added and the organic phase separated, concentrated at reduced pressure and then dried under vacuum. Polymer yield: 7.29 g. GPC analysis: Mn = 1350, Mw = 14800, PD = 10.94 (PS STD., bimodal distribution).

### EXAMPLE 12

### Preparation of 10 wt% Y loaded zeolite HY for THF polymerization

50 g of zeolite LZY-82 (NH₄⁺ ion form of zeolite Y) was slurried into 1 liter distilled water and the pH was adjusted to 4 with nitric acid. 22.0 g yttrium nitrate pentahydrate (calculated to give a final product having ∼10 wt% Y) was added and the slurry stirred and warmed for 4 hours. The stirring was stopped and the mixture left to sit overnight to complete the exchange. The solid was collected by filtration and washed with 1 liter distilled water then suction dried. The wet solid was loaded into a horizontal tube furnace and fired as follows in flowing dry air (flow rate 200 mL/min) :
Room temperature to 500°C at 12°C/min.
Hold at 500°C for 2 hours.
Cool to room temperature over 1 hour and collect white powder.

The cool powder was quickly transferred to a tightly capped sample vial in order to minimize exposure to atmospheric moisture. A small portion of the material was sent for x-ray diffraction analysis and showed that the zeolite crystallinity had been maintained during the ion-exchange and calcination procedure and that there was no presence of a bulk Y₂O₃ phase.

### EXAMPLE 13

### Polymerization of THF with 10 wt% Yttrium Zeolite HY and Acetic Anhydride

In a dry box, the 10% yttrium zeolite HY of Example 12 (10.00 g) was added to an oven dried 100 mL round bottom flask equipped with a stirring bar. The flask was sealed with a rubber septum and removed from the dry box. A nitrogen bleed was attached and THF (20.00 mL) and acetic anhydride (1.00 mL) were added. After 4 hrs. ether (50 mL) was added to the resulting polymerized solution. After filtering, the organic phase was washed with 5% NaOH (10 mL), separated concentrated at reduced pressure then dried under vacuum. Polymer yield: 6.11 g. GPC analysis: Mn = 690, Mw = 4120, PD = 10.94 (PS STD., bimodal distribution).

### EXAMPLE 14

### Preparation of Y loaded zeolite mordenite for THF polymerization

10 g of zeolite H-mordenite was slurried into 1 liter distilled water and the pH was adjusted to 4 with nitric acid. 2.20 g yttrium nitrate pentahydrate (calculated to give a final product having ∼5 wt% Y) was added and the slurry stirred and warmed for 4 hours.
The stirring was stopped and the mixture left to sit overnight to complete the exchange. The solid was collected by filtration and washed with 1 liter distilled water then suction dried. The wet solid was loaded into a horizontal tube furnace and fired as follows in flowing dry air (flow rate 200 mL/min) :
Room temperature to 500°C at 12°C/min.
Hold at 500°C for 2 hours.
Cool to room temperature over 1 hour and collect white powder.

The cool powder was quickly transferred to a tightly capped sample vial in order to minimize exposure to atmospheric moisture. A small portion of the material was sent for x-ray diffraction analysis and showed that the zeolite crystallinity had been maintained during the ion-exchange and calcination procedure and that there was no presence of a bulk Y₂O₃ phase.

### EXAMPLE 15

### Polymerization of THF with Yttrium Mordenite and Acetic Anhydride

In a dry box, the yttrium mordenite of Example 14 (5.00 g) was added to an oven dried 100 mL round bottom flask equipped with a stirring bar. The flask was sealed with a rubber septum and removed from the dry box. A nitrogen bleed was attached and THF (20.00 mL) and acetic anhydride (1.00 mL) were added. After stirring overnight ether (50 mL) was added to the resulting polymerized solution. After filtering, ether (50 mL) and water (25 mL) were added and the organic phase separated, concentrated at reduced pressure and then dried under vacuum. Polymer yield: 1.43 g. GPC analysis: Mn = 6020, Mw = 15500, PD = 2.58 (PS STD.).

### EXAMPLE 16

### Preparation of ∼10 wt% Didymium loaded zeolite HY for THF polymerization

50 g of zeolite LZY-82 (NH₄⁺ ion form of zeolite Y) was slurried into 1 liter distilled water and the pH was adjusted to 4 with nitric acid. 10 g didymium chloride was added and the slurry stirred and warmed for 1 hour. The zeolite was recovered by filtration washed with 1 liter distilled water and then re-slurried into 1 liter fresh distilled water. Another 10 g didymium chloride was added and the pH adjusted to 4 with nitric acid and this slurry was stirred for 1 hour. The stirring was stopped and the mixture left to sit overnight to complete the exchange. The solid was collected by filtration and washed with 1 liter distilled water then suction dried. The wet solid was loaded into a horizontal tube furnace and fired as follows in flowing dry air (flow rate 200 mL/min) :
Room temperature to 500°C at 12°C/min.
Hold at 500°C for 2 hours.
Cool to room temperature over 1 hour and collect white powder.

The cool powder was quickly transferred to a tightly capped sample vial in order to minimize exposure to atmospheric moisture. The powder was then pressed to 10 tons and the resultant cake sieved through 10-20 mesh to give granulated catalyst.

### EXAMPLE 17

### Polymerization of THE with 10% Didymium Zeolite HY and Acetic Anhydride

In a dry box, the 10% Didymium zeolite HY of Example 16 (10.00 g) was added to an oven dried 100 mL round bottom flask equipped with a stirring bar. The flask was sealed with a rubber septum and removed from the dry box. A nitrogen bleed was attached and THF (20.00 mL) and acetic anhydride (1.00 mL) were added. After stirring overnight the polymerized solution was fitered, then ether (25 mL), THF (25 mL) and water (25 mL) were added the organic phase separated concentrated at reduced pressure then dried under vacuum. Polymer yield: 7.56 g. GPC analysis: Mn = 1010, Mw = 7150, PD = 7.08 (PS STD., bimodal distribution).

### EXAMPLE 18

### Preparation of 5 wt% Sc loaded zeolite HY for THF polymerization

5 g of zeolite LZY-82 (NH₄⁺ ion form of zeolite Y) was slurried into 1 liter distilled water and the pH was adjusted to 4 with nitric acid. 1.44 g scandium chloride (calculated to give a final product having ∼5 wt% Sc) was added and the slurry stirred and warmed for 4 hours. The stirring was stopped and the mixture left to sit overnight to complete the exchange. The solid was collected by filtration and washed with 1 liter distilled water then suction dried. The wet solid was loaded into a horizontal tube furnace and fired as follows in flowing dry air (flow rate 200 mL/min) :
Room temperature to 500°C at 12°C/min.
Hold at 500°C for 2 hours.
Cool to room temperature over 1 hour and collect white powder.

The cool powder was quickly transferred to a tightly capped sample vial in order to minimize exposure to atmospheric moisture. A small portion of the material was sent for x-ray diffraction analysis and showed that the zeolite crystallinity had been maintained during the ion-exchange and calcination procedure and that there was no presence of a bulk Sc₂O₃ phase.

### EXAMPLE 19

### Polymerization of THF with 5% Scandium Zeolite HY and Acetic Anhydride

In a dry box, the 5% scandium zeolite HY of Example 18 (3.94 g) was added to an oven dried 100 mL round bottom flask equipped with a stirring bar. The flask was sealed with a rubber septum and removed from the dry box. A nitrogen bleed was attached and THF (20.00 mL) and acetic anhydride (1.00 mL) were added. After stirring overnight the polymerized solution was filtered, then ether (25 mL), THF (25 mL) and water (25 mL) were added the organic phase separated concentrated at reduced pressure then dried under vacuum. Polymer yield: 4.48 g. GPC analysis: Mn = 736, Mw = 7890, PD = 10.72 (PS STD., bimodal distribution).

### EXAMPLE 20

### Preparation of 10 wt% Y on zeolite NaY pellets

12.5 g yttrium nitrate pentahydrate was dissolved in 1liter distilled water and the pH adjusted to 4 with nitric acid. 25 g zeolite LZY-52 (NaY) pellets (3 mm dia x 3 mm) was added and the mixture allowed to sit overnight. The pellets were then filtered and washed with 1liter distilled water and suction dried. The pellets were then calcined in flowing dry air (flow rate 200 mL/min) :
Room temperature to 500°C at 12°C/min.
Hold at 500°C for 2 hours.
Cool to room temperature over 1 hour and collect white powder.

The cool pellets were quickly transferred to a tightly capped sample vial in order to minimize exposure to atmospheric moisture.

### EXAMPLE 21

### Polymerization of THF with 10% Yttrium Triflate on Zeolite NaY Pellets and Acetic Anhydride

In a dry box, the 10% yttrium triflate on zeolite NaY pellets of Example 20 (10.00 g) was added to an oven dried 100 mL round bottom flask equipped with a stirring bar. The flask was sealed with a rubber septum and removed from the dry box. A nitrogen bleed was attached and THF (20.00 mL) and acetic anhydride (1.00 mL) were added. After stirring for 1 hour THF (50 mL) the polymerized solution then filtered. The fitrate was washed with water (25 mL), then ether (50 mL) was added. The organic phase was separated concentrated at reduced pressure then dried under vacuum. Polymer yield: 0.700 g. GPC analysis: Mn = 18800, Mw = 27100, PD = 1.44 (PS STD.).

### EXAMPLE 22

### Preparation of Yttrium triflate supported on alumina derivatized with diethylmalonate

95 mL ethanol and 5 mL water were mixed and the pH adjusted to 5 with acetic acid. 2 g DETM was added and the mix stirred for 5 minutes. 10 g γ-alumina was added and the slurry stirred a further 3 min. The solid was allowed to settle and the supernatant liquid decanted. The recovered solid was then washed with two portions of 25 mL ethanol and suction dried. The solid was finally dried in flowing nitrogen (200 mL/min) by heating to 110°C and holding at that temperature for 1 hour. The cooled powder was immediately transferred to a nitrogen glove box.

Under a dry nitrogen atmosphere inside a glove box, 0.5 g yttrium triflate was dissolved in 25 mL acetonitrile and 5 g of the alumina powder prepared above was added. The slurry was stirred for 1 hour and then filtered, rinsed with 25 mL acetonitrile and suction dried. The powder was then pumped to dryness in vacuum and stored under nitrogen.

### EXAMPLE 23

### Polymerization of THF with Yttrium Triflate Supported on Alumina Derivatized with Diethyl Malonate

In a dry box, the yttrium triflate supported on diethylmalonate derivatized alumina of Example 22 (5.00 g) was weighed in an oven dried 100 mL RB flask. The flask was sealed with a rubber septum and removed from the dry box. A nitrogen purge was attached and THF (15.00 mL) and acetic anhydride (2.00 mL) added via syringe. After 360 minutes the polymerization was terminated by decanting, followed by concentration at reduced pressure and then drying under vacuum. Polymer yield: 2.53 g. GPC analysis: Mn = 8300, Mw = 20900, PD = 2.52 (PS STD.).

### EXAMPLE 24

### Preparation of bis(n-cyclopentadienyl)tetra-hydrofuran-bis(trifluoromethanesulfonato)-zirconium supported on silica derivatized with DETM

95 mL ethanol and 5 mL water were mixed and the pH adjusted to 5 with acetic acid. 2 g DETM was added and the mix stirred for 5 minutes. 10 g silica was added and the slurry stirred a further 3 mins. The solid was allowed to settle and the supernatant liquid decanted. The recovered solid was then washed with two portions of 25 mL ethanol and suction dried. The solid was finally dried in flowing nitrogen (200 mL/min) by heating to 110°C and holding at that temperature for 1 hour. The cooled powder was immediately transferred to a nitrogen glove box.

Under a dry nitrogen atmosphere inside a glove box, 0.5 g bis(n-cyclopentadienyl)tetrahydrofuran-bis(tri-fluoromethanesulfonato)zirconium was dissolved in 25 mL acetonitrile and 5 g of the silica powder was added. The slurry was stirred for 1 hour and then filtered, rinsed with 25 mL acetonitrile and suction dried. The powder was then pumped to dryness in vacuum and stored under nitrogen.

### EXAMPLE 25

### Polymerization of THF with Bis(n-cyclopentadienyl)tetrahydrofuran-bis(trifluoromethanesulfonato)Zirconium Supported on Silica Derivatized with Diethyl Malonate

In a dry box, the bis(n-cyclopentadienyl)tetra-hydrofuran-bis(trifluoromethane-sulfonato)zirconium supported on silica of Example 24 (5.00 g) was weighed in an oven dried 100 mL RB flask. The flask was sealed with a rubber septum and removed from the dry box. A nitrogen purge was attached and THF (15.00 mL) and acetic anhydride (2.00 mL) were added via syringe. After 360 minutes the polymerization was terminated by decanting, followed by concentration at reduced pressure and then drying under vacuum. Polymer yield: 2.05 g. GPC analysis: Mn = 7620, Mw = 19000, PD = 2.29 (PS STD.).

### EXAMPLE 26

### Preparation of bis(n-cyclopentadienyl)tetra-hydrofuran-bis(trifluoromethanesulfonato)zirconium supported on alumina derivatized with DETM

95mL ethanol and 5 mL water were mixed and the pH adjusted to 5 with acetic acid. 2 g DETM was added and the mix stirred for 5 minutes. 10 g λ-alumina was added and the slurry stirred a further 3 mins. The solid was allowed to settle and the supernatant liquid decanted. The recovered solid was then washed with two portions of 25 mL ethanol and suction dried. The solid was finally dried in flowing nitrogen (200 mL/min) by heating to 110°C and holding at that temperature for 1 hour. The cooled powder was immediately transferred to a nitrogen glove box.

Under a dry nitrogen atmosphere inside a glove box, 0.5 g bis(n-cyclopentadienyl)tetrahydrofuran-bis(tri-fluoromethanesulfonato)zirconium was dissolved in 25 mL acetonitrile and 5 g of the alumina powder prepared above was added. The slurry was stirred for 1 hour and then filtered, rinsed with 25 mL acetonitrile and suction dried. The powder was then pumped to dryness in vacuum and stored under nitrogen.

### EXAMPLE 27

### Polymerization of THE with Bis(n-cyclopentdienyl)tetrahydrofuran-bis(trifluoromethanesulfonato)Zirconium Supported on Alumina Derivatized with Diethyl Malonate

In a dry box, the bis(n-cyclopentdienyl)tetrahydrofuran-bis(trifluoromethanesulfonato)zirconium supported on alumina of Example 26 (5.00 g) was weighed in an oven dried 100 mL RB flask. The flask was sealed with a rubber septum and removed from the dry box. A nitrogen purge was attached and THF (15.00 mL) and acetic anhydride (2.00 mL) added via syringe. After 360 minutes the polymerization was terminated by decanting, followed by concentration at reduced pressure and then drying under vacuum. Polymer yield: 1.99 g. GPC analysis: Mn = 9600, Mw = 25800, PD = 2.69 (PS STD.).

### EXAMPLE 28

### Preparation of ∼10 wt% Ytterbium triflate supported on silica-alumina

50 g silica-alumina pellets (3.2 mm dia. x 3.2 mm) were placed in 250 mL distilled water. The pH was adjusted to 3 with acetic acid and the slurry was stirred for 15 mins. The pellets were collected by filtration and then added to a solution of 190 mL ethanol, 10 mL water in which the pH was adjusted to 5 with acetic acid and then 5 g DETM was added. Stirred for 30 min and then the supernatant liquid was decanted. The pellets were washed with two portions of 25 mL ethanol and then suction dried prior to drying in flowing nitrogen (200 mL/min) at 110°C for 1 hour. The pellets were then transferred to a nitrogen glove box. Inside the glove box 10 g ytterbium triflate was dissolved in 100 mL dry acetonitrile and this solution was added to the dry pellets. The slurry then sat overnight under nitrogen before being evaporated to dryness. The recovered solid was washed with three 25 mL portions of dry acetonitrile, suction dried and then dried in flowing nitrogen to 110°C for 1 hour. The dry pellets were stored under nitrogen in a glove box.

### EXAMPLE 29

### Depolymerization of Polytetrahydrofuran (Terathane® 1000) with 10% Ytterbium Triflate Supported on Silica-Alumina

Polytetrahydrofuran with hydroxyl ends (Terathane® 1000, 300 g, Aldrich) and the 10% ytterbium triflate supported on silica-alumina of Example 28 (10 g) were placed in a 500 mL three neck flask equipped with a stirring bar, Vigreaux column (12") and a fractional distillation head. A nitrogen purge was attached and all other openings were glass stoppered. The resulting mixture was heated with an oil bath and the resulting water clear distillate fractions collected as follows:

| Fraction | Oil Bath Temo (°C) | Rxn Temp (°C) | Head Temp (°C) | Weight (g) |
|---|---|---|---|---|
| 1 | 180 | 152 | 64 | 70.72 |
| 2 | 198 | 163 | 66 | 112.0 |
| 3 | 196 | 124 | 66 | 59.00 |
| 4 | 202 | 149 | 66 | 55.00 |

Total weight of distillate collected: 296.72 g
% Yield (Recovery): 98.9%
GC analyses of the various fractions confirm the product to be THF

### COMPARATIVE EXAMPLE 1

In a dry box, zeolite HY (1.00 g) was added to an oven dry 100 mL RB flask equipped with a stirring bar. The flask was sealed with a rubber septum and removed from the dry box. After the attachment of a nitrogen bleed THF (20.00 mL) was added by syringe. After 45 minutes acetic anhydride (1.00 mL) was added. After filtering off the zeolite catalyst and concentration of the filtrate, no polymer was obtained.

## Claims

1. A process for the polymerization of cyclic ethers, comprising, contacting one or more oxiranes, oxetanes, tetrahydrofurans, oxepanes, 1,3-dioxolanes, or 1,3,5-trioxanes; with a heterogeneous catalyst containing a metal perfluoroalkylsulfonate attached to the surface of said catalyst, said metal selected from the group consisting of strontium, barium, scandium, yttrium, the rare earth metals, titanium, zirconium, hafnium, vanadium, niobium, chromium, molybdenum, tungsten, tantalum, rhenium, iron, ruthenium, osmium, rhodium, iridium, palladium, copper, platinum, silver, gold, zinc, cadmium, mercury, aluminum, gallium, indium, thulium, germanium, tin, lead, arsenic, antimony, mischmetall and bismuth; and an accelerator selected from the group consisting of carboxylic anhydrides, acyl halides, and carboxylic acids whose pKa in water is less than about 6.

2. The process as recited in claim 1 wherein said cyclic ether is one or more of tetrahydrofurans, oxepanes, 1,3-dioxolanes, or 1,3,5-trioxanes.

3. The process as recited in claim I wherein said cyclic ether comprises the formula: 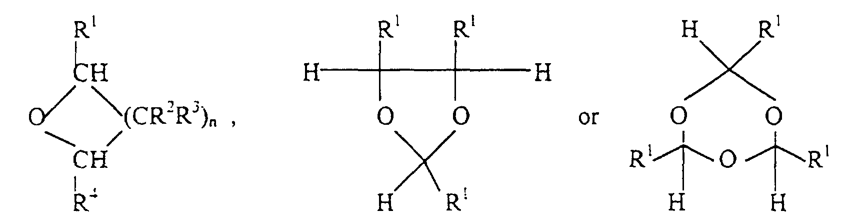 wherein n is 2 or 4, and each R¹, R², R³ and R⁴ is independently hydrogen or hydrocarbyl containing 1 to 20 carbon atoms.

4. The process as recited in claim 3 wherein all of R¹, R², R³ and R⁴ are hydrogen.

5. The process as recited in claim 3 wherein n is 2; and wherein R¹, one of R², both of R³ and R⁴ are hydrogen and the remaining R² is alkyl containing 1-4 carbon atoms.

6. The process as recited in claim 1 wherein said metal is strontium, scandium, yttrium, the rare earth metals, titanium, zirconium, hafnium, vanadium, niobium, tantalum, chromium, molybdenum, tungsten, rhenium, iron, ruthenium, palladium, copper, gold, zinc, tin or bismuth.

7. The process as recited in claim 6 wherein said metal is yttrium, ytterbium, dysprosium, erbium, neodymium, lanthanum, mischmetall, scandium or zirconium.

8. The process as recited in claim 1 wherein said accelerator is a carboxylic acid whose pKa in water is about 6 or less.

9. The process as recited in claim 1 wherein said heterogeneous catalyst support is alumina, silica, silica-aluminate, carbon or zirconia.

10. The process as recited in claim 1 wherein the metal perfluoroalkylsulfonate is not attached to the surface of the catalyst through the perfluoroalkylsulfonate anion.

11. The process as recited in claim 1 in which the metal is other than silver.

## Patentansprüche

1. Verfahren für die Polymerisation cyclischer Ether, umfassend Kontaktieren von einem oder mehreren Oxiranen, Oxetanen, Tetrahydrofuranen, Oxepanen, 1,3-Dioxolanen oder 1,3,5-Trioxanen mit einem heterogenen Katalysator, enthaltend ein Metallperfluoralkylsulfonat, das an der Oberfläche des Katalysators angebracht ist, wobei das Metall ausgewählt ist aus der Gruppe, bestehend aus: Strontium, Barium, Scandium, Yttrium, den Seltenerdmetallen, Titan, Zirconium, Hafnium, Vanadium, Niob, Chrom, Molybdän, Wolfram, Tantal, Rhenium, Eisen, Ruthenium, Osmium, Rhodium, Iridium, Palladium, Kupfer, Platin, Silber, Gold, Zink, Cadmium, Quecksilber, Aluminium, Gallium, Indium, Thulium, Germanium, Zinn, Blei, Arsen, Antimon, Mischmetall und Bismut; sowie einen Beschleuniger, ausgewählt aus der Gruppe, bestehend aus Carbonsäureanhydriden, Acylhalogeniden und Carbonsäuren, deren pKa-Wert in Wasser kleiner ist als etwa 6.

2. Verfahren nach Anspruch 1, bei welchem der cyclische Ether einer oder mehrere von Tetrahydrofuranen, Oxepanen, 1,3-Dioxolanen oder 1,3,5-Trioxanen ist.

3. Verfahren nach Anspruch 1, bei welchem der cyclische Ether die Formel hat: 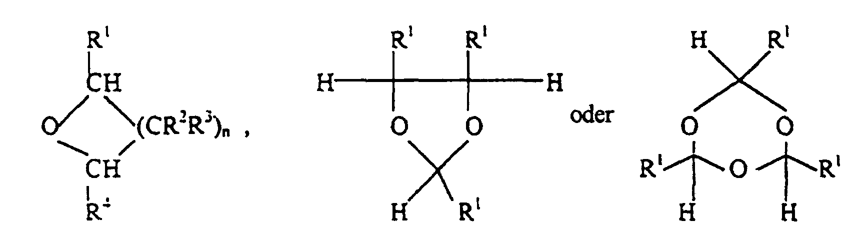 worin n 2 oder 4 ist und jedes R¹, R², R³ und R⁴ unabhängig Wasserstoff oder Hydrocarbyl ist, das 1 bis 20 Kohlenstoffatome enthält.

4. Verfahren nach Anspruch 3, bei welchem alle R¹, R², R³ und R⁴ Wasserstoff sind.

5. Verfahren nach Anspruch 3, bei welchem n 2 ist und worin R¹, eines von R², beide R³ und R⁴ Wasserstoff sind und das übrige R² Alkyl ist, das 1 bis 4 Kohlenstoffatome enthält.

6. Verfahren nach Anspruch 1, bei welchem das Metall Strontium ist, Scandium, Yttrium, die Seltenerdmetalle, Titan, Zirconium, Hafnium, Vanadium, Niob, Tantal, Chrom, Molybdän, Wolfram, Rhenium, Eisen, Ruthenium, Palladium, Kupfer, Gold, Zink, Zinn oder Bismut.

7. Verfahren nach Anspruch 6, bei welchem das Metall Yttrium ist, Ytterbium, Dysprosium, Erbium, Neodym, Lanthan, Mischmetall, Scandium oder Zirconium.

8. Verfahren nach Anspruch 1, bei welchem der Beschleuniger eine Carbonsäure ist, deren pKa-Wert in Wasser etwa 6 oder weniger beträgt.

9. Verfahren nach Anspruch 1, bei welchem der heterogene Katalysatorträger Aluminiumoxid, Siliciumdioxid, Siliciumdioxid-Aluminat, Kohlenstoff oder Zirconiumdioxid ist.

10. Verfahren nach Anspruch 1, bei welchem das Metallperfluoralkylsulfonat nicht an der Oberfläche des Katalysators über das Perfluoralkylsulfonat-Ion angebracht ist.

11. Verfahren nach Anspruch 1, bei welchem das Metall ein anderes als Silber ist.

## Revendications

1. Procédé pour la polymérisation d'éthers cycliques, comprenant la mise en contact d'un ou de plusieurs oxyrannes, oxétannes, tétrahydrofurannes, oxépannes, 1,3-dioxolannes ou 1,3,5-trioxannes; avec un catalyseur hétérogène contenant un perfluoroalkylsulfonate de métal attaché à la surface dudit catalyseur, ledit métal étant choisi dans le groupe constitué de strontium, de baryum, de scandium, d'yttrium, de métaux des terres rares, de titane, de zirconium, d'hafnium, de vanadium, de niobium, de chrome, de molybdène, de tungstène, de tantale, de rhénium, de fer, de ruthénium, d'osmium, de rhodium, d'iridium, de palladium, de cuivre, de platine, d'argent, d'or, de zinc, de cadmium, de mercure, d'aluminium, de gallium, d'indium, de thulium, de germanium, d'étain, de plomb, d'arsenic, d'antimoine, de mischmétal et de bismuth; et un accélérateur choisi dans le groupe constitué d'anhydrides carboxyliques, d'halogénures d'acyle et d'acides carboxyliques dont le pKa dans l'eau est inférieur à environ 6.

2. Procédé suivant la revendication 1, dans lequel ledit éther cyclique est un ou plusieurs tétrahydrofurannes, oxépannes, 1,3-dioxolannes ou 1,3,5-trioxannes.

3. Procédé suivant la revendication 1, dans lequel ledit éther cyclique comprend la formule: 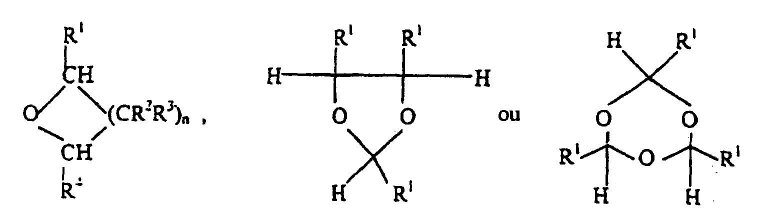 dans laquelle n est 2 ou 4, et chaque R¹, R², R³ et R⁴ est indépendamment un hydrogène ou un groupe hydrocarbyle contenant de 1 à 20 atomes de carbone.

4. Procédé suivant la revendication 3, dans lequel tous les radicaux R¹, R², R³ et R⁴ sont des hydrogènes.

5. Procédé suivant la revendication 3, dans lequel n est 2; et dans lequel R¹, un des R², à la fois R³ et R⁴ sont des hydrogènes et le R² restant est un groupe alkyle contenant 1-4 atomes de carbone.

6. Procédé suivant la revendication 1, dans lequel ledit métal est le strontium, le scandium, l'yttrium, des métaux des terres rares, le titane, le zirconium, l'hafnium, le vanadium, le niobium, le tantale, le chrome, le molybdène, le tungstène, le rhénium, le fer, le ruthénium, le palladium, le cuivre, l'or, le zinc, l'étain ou le bismuth.

7. Procédé suivant la revendication 6, dans lequel ledit métal est l'yttrium, l'ytterbium, le dysprosium, l'erbium, le néodyme, le lanthane, un mischmétal, le scandium ou le zirconium.

8. Procédé suivant la revendication 1, dans lequel ledit accélérateur est un acide carboxylique dont le pKa dans l'eau est inférieur ou égal à environ 6.

9. Procédé suivant la revendication 1, dans lequel ledit support de catalyseur hétérogène est une alumine, une silice, un silico-aluminate, un carbone ou une zircone.

10. Procédé suivant la revendication 1, dans lequel le perfluoroalkylsulfonate de métal n'est pas attaché à la surface du catalyseur par l'anion de perfluoroalkylsulfonate.

11. Procédé suivant la revendication 1, dans lequel le métal est différent de l'argent.
